# EUROPÄISCHE PATENTANMELDUNG

(11) **EP 2 198 972 A1**
(43) Veröffentlichungstag der Anmeldung: **23.06.2010**
(21) Anmeldenummer: 08021994.2
(22) Anmeldetag: 18.12.2008
(51) Int. Cl.: B05B 11/00, B65D 1/02, B65D 83/00, A61M 11/00, B29C 47/06

(54) **Reservoir und Zerstäuber**

(71) Anmelder: Boehringer Ingelheim International GmbH, 55216 Ingelheim am Rhein (DE)
(72) Erfinder: Huhs, Volker, 55216 Ingelheim am Rhein (DE); Hahn, Christoph, 55216 Ingelheim am Rhein (DE); Mathe, Gerald, 55216 Ingelheim am Rhein (DE)
(74) Vertreter: Gesthuysen, von Rohr & Eggert

(57) **Zusammenfassung**

Es werden ein Reservoir (3) für einen Zerstäuber (1), ein Zerstäuber (1) und ein Verfahren zur Herstellung eines Reservoirs (3) vorgeschlagen. Das Reservoir (3) weist eine Außenhülle (23) auf, die direkt auf eine Wandung (25) eines Innenbeutels extrudiert wird, wobei die Wandung (25) von der Außenhülle (23) lösbar ist.

## Beschreibung

Die vorliegende Erfindung betrifft ein Reservoir gemäß dem Oberbegriff des Anspruchs 1, einen Zerstäuber gemäß dem Oberbegriff des Anspruchs 12 sowie ein Verfahren zum Herstellen eines Reservoirs gemäß dem Oberbegriff des Anspruchs 13.

Aus der WO 96/06011 A1 und der WO 00/49988 A2 sowie der WO 99/43571 A1 ist jeweils ein Behälter für einen Zerstäuber bzw. Inhalator bekannt. Der Behälter weist eine starre metallische Außenhülle und einen darin aufgenommenen Beutel auf. Der Beutel bildet einen Fluidraum für eine Arzneimittelzubereitung und kollabiert, wenn die Arzneimittelzubereitung entnommen wird. Die Herstellung des bekannten Behälters ist aufwendig, insbesondere wenn die Hülle durch Tiefziehen hergestellt wird.

Der vorliegenden Erfindung liegt die Aufgabe zugrunde, ein Reservoir mit einem Fluid, insbesondere einer Arzneimittelzubereitung, einen Zerstäuber mit einem Reservoir und ein Verfahren zum Herstellen eines Reservoirs anzugeben, wobei eine einfache, schnelle und/oder kostengünstige Herstellung ermöglicht wird und/oder eine Außenhülle aus Kunststoff auf einfache Weise gebildet wird.

Die obige Aufgabe wird durch ein Reservoir gemäß Anspruch 1, einen Zerstäuber gemäß Anspruch 12 oder ein Verfahren gemäß Anspruch 13 gelöst, Vorteilhafte Weiterbildungen sind Gegenstand der Unteransprüche.

Ein Aspekt der vorliegenden Erfindung liegt darin, daß das Reservoir mit einer zumindest im wesentlichen starren Außenhülle versehen wird, die auf die flexible bzw. verformbare, einen Fluidraum für das Fluid zumindest teilweise begrenzende Wandung des Reservoirs extrudiert ist, wobei die Wandung zumindest partiell von der Außenhülle lösbar ist. Dies gestattet eine einfache, schnelle und/oder kostengünstige Herstellung sowie die Bildung der Außenhülle aus Kunststoff.

Unter dem Begriff "Extrudieren" ist hier vorzugsweise ein Spritzen bzw. Spritzgießen der Außenhülle auf die Wandung zu verstehen. Insbesondere bildet die Wandung also eine Formfläche für die Außenhülle bei deren Herstellung.

Unter dem Begriff "Extrudieren" ist in einem engeren Sinne vorzugsweise die Herstellung der Außenhülle in einem Endlosverfahren bzw. kontinuierlich und/oder die Erzeugung der Außenhülle über eine Flach- oder Hohldüse und/oder ein entsprechendes Anformen zu verstehen. In einem weiteren Sinne soll unter dem Begriff "Extrudieren" aber auch ein sonstiges Anformen, Anspritzen bzw. Spritzgießen der Außenhülle auf die Wandung, ggf. auch diskontinuierlich, zu verstehen sein.

Weitere Vorteile, Merkmale, Eigenschaften und Aspekte der vorliegenden Erfindung ergeben sich aus den Ansprüchen und der folgenden Beschreibung einer bevorzugten Ausführungsform anhand der Zeichnung. Es zeigt:
- Fig. 1: einen schematischen Schnitt eines vorschlagsgemäßen Zerstäubers im ungespannten Zustand;
- Fig. 2: einen schematischen, um 90° gegenüber Fig. 1 gedrehten Schnitt des Zerstäubers im gespannten Zustand;
- Fig. 3: einen schematischen Schnitt eines vorschlagsgemäßen Reservoirs gemäß einer zweiten Ausführungsform und
- Fig. 4: einen auschnittsweisen schematischen Schnitt eines Wandlaufs des Reservoirs.

In den Figuren werden für gleiche oder ähnliche Teile dieselben Bezugszeichen verwendet, wobei insbesondere entsprechende oder vergleichbare Eigenschaften und Vorteile erreicht werden, auch wenn eine wiederholte Beschreibung weggelassen ist.

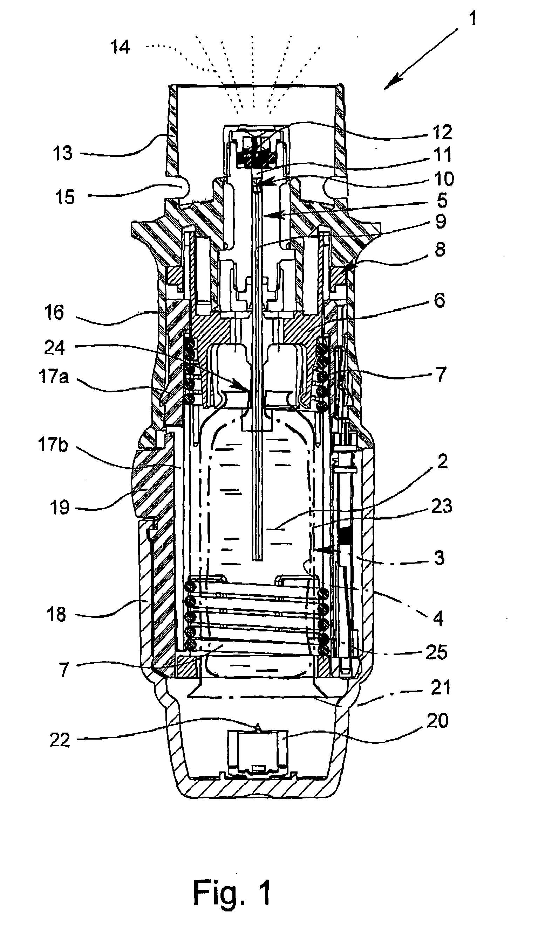

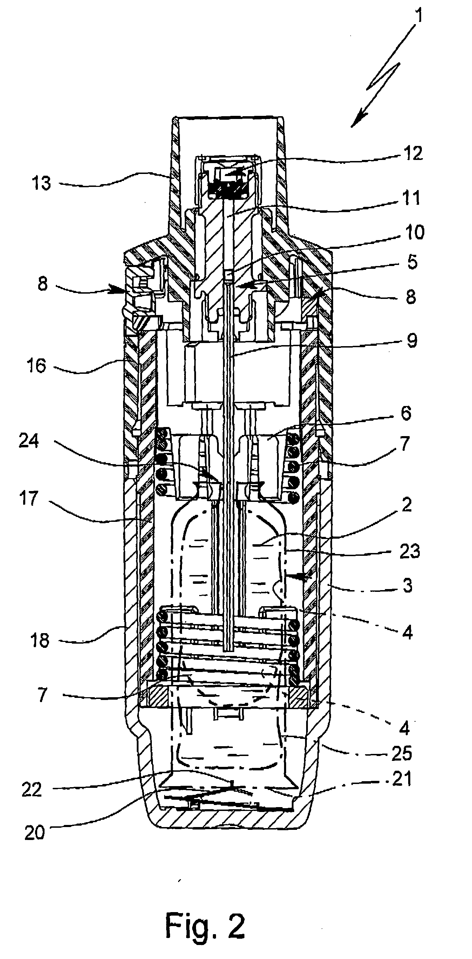

Fig. 1 und 2 zeigen einen vorschlagsgemäßen Zerstäuber 1 zur Zerstäubung eines Fluids 2, insbesondere einer Flüssigkeit bzw. Arzneimittelzubereitung, in einer schematischen Darstellung im ungespannten Zustand (Fig. 1) und gespannten Zustand (Fig. 2). Der Zerstäuber 1 ist insbesondere als tragbarer Inhalator ausgebildet und/oder arbeitet vorzugsweise ohne Treibgas.

Bei Zerstäubung des Fluids 2 bzw. der Arzneimittelzubereitung wird vorzugsweise ein lungengängiges Aerosol 14 (Fig. 1) gebildet, das von einem nicht dargestellten Benutzer bzw. Patienten aufgenommen, insbesondere eingeatmet bzw. inhaliert werden kann. Üblicherweise erfolgt das Inhalieren wenigstens einmal täglich, insbesondere mehrmals täglich, vorzugsweise in vorbestimmten Zeitabständen, insbesondere in Abhängigkeit von der Erkrankung des Patienten.

Der Zerstäuber 1 weist ein vorzugsweise einsetzbares und ggf. wechselbares Reservoir 3 mit dem Fluid 2 auf, wie in Fig. 1 und 2 gezeigt. Vorzugsweise enthält das Reservoir 3 eine ausreichende Menge (typischerweise 2 bis 10 oder 2 bis 15 ml) an Fluid 2 bzw. Wirkstoff für mehrere Dosen, um also mehrere Zerstäubungen oder Anwendungen zu ermöglichen.

Das Reservoir 3 ist vorzugsweise im wesentlichen zylindrisch bzw, kartuschenartig, länglich und/oder als insbesondere starrer Behälter ausgebildet und/oder beispielsweise von unten, nach Öffnen des Zerstäubers 1, in diesen einsetzbar und ggf. wechselbar.

Das Reservoir 3 weist einen vorzugsweise beutelartig oder schlauchartig ausgebildeten Fluidraum 4 mit dem Fluid 2 auf. Der Fluidraum 4 bzw. eine den Fluidraum 4 begrenzende Wandung 25 ist vorzugsweise - zumindest bereichsweise - flexibel, verformbar und/oder kollabierbar ausgebildet.

Der Zerstäuber 1 weist vorzugsweise eine Fördereinrichtung, insbesondere einen Druckerzeuger 5, zur Förderung und/oder Zerstäubung des Fluids 2, insbesondere jeweils in einer vorbestimmten, ggf. einstellbaren Dosiermenge auf.

Der Zerstäuber 1 bzw. Druckerzeuger 5 weist insbesondere eine Halterung 6 für das Reservoir 3, eine zugeordnete, nur teilweise dargestellte Antriebsfeder 7 vorzugsweise mit einem zugeordneten, zur Entsperrung manuell betätigbaren Sperrelement 8, ein vorzugsweise als Kapillare ausgebildetes Förderelement bzw, Förderrohr 9, ein optionales Ventil, insbesondere Rückschlagventil 10, eine Druckkammer 11 und/oder eine Austragsdüse 12 insbesondere im Bereich eines Mundstücks 13 oder sonstigen Endstücks auf.

Das Reservoir 3 wird über die Halterung 6, insbesondere klemmend oder rastend, so in dem Zerstäuber 1 fixiert, daß das Förderelement in den Fluidraum 4 eintaucht und/oder damit fluidisch verbunden wird. Die Halterung 6 kann dabei derart ausgebildet sein, daß das Reservoir 3 ausgetauscht werden kann.

Beim axialen Spannen der Antriebsfeder 7 wird die Halterung 6 mit dem Reservoir 3 und dem Förderelement bei den Darstellungen nach unten bewegt und das Fluid 2 - genauer gesagt die nächste Dosis - aus dem Reservoir 3 über das Rückschlagventil 10 in die Druckkammer 11 des Druckerzeugers 5 gesaugt. Der Fluidraum 4 (Beutel) kollabiert in Abhängigkeit von der Entnahme von Fluid 2, wie beispielsweise und nur schematisch durch die gestrichelte Linie im unteren Bereich des Fluidraums 4 in Fig. 2 angedeutet.

Beim anschließenden Entspannen der Antriebsfeder 7 nach Betätigung des Sperrelements 8 zur Zerstäubung wird das Fluid 2 in der Druckkammer 11 unter Druck gesetzt, indem das Förderelement bei nun geschlossenem Rückschlagventil 10 vorzugsweise allein durch die Kraft der Antriebsfeder 7 wieder nach oben bewegt wird und nun als Druckstempel wirkt. Dieser Druck treibt das Fluid 2 durch die Austragsdüse 12 aus, wobei es in das vorzugsweise lungengängige Aerosol 14 zerstäubt wird, wie in Fig. 1 angedeutet.

Der nicht dargestellte Benutzer bzw. Patient kann das Aerosol 14 inhalieren, wobei vorzugsweise Zuluft über mindestens eine Zuluftöffnung 15 in das Mundstück 13 saugbar ist.

Während des Zerstäubungsvorgangs bzw. -hubs wird das Reservoir 3 von der Antriebsfeder 7 wieder in seine Ausgangslage zurückbewegt. Das Reservoir 3 führt also vorzugsweise eine Hubbewegung während des Spannvorgangs und während des Zerstäubungsvorgangs aus.

Anstelle des Druckerzeugers 5 und/oder der Antriebsfeder 7 können auch sonstige Mittel und/oder Einrichtungen eingesetzt werden.

Der Zerstäuber 1 weist insbesondere ein erstes Gehäuseteil (Oberteil) 16 und ein demgegenüber drehbares Innenteil 17 (Fig. 2) mit einem oberen Teil 17a und einem unteren Teil 17b (Fig. 1) auf, wobei an dem Innenteil 17 ein insbesondere manuell betätigbares oder drehbares, zweites Gehäuseteil (Unterteil) 18 vorzugsweise mittels eines Sicherheitsverschlusses bzw. Halteelements 19 lösbar befestigt, insbesondere darauf aufgesteckt, ist. Insbesondere ist der Sicherheitsverschluß bzw. das Halteelement 19 derart ausgebildet, daß ein versehentliches Öffnen des Zerstäubers 1 bzw. Abziehen des zweiten Gehäuseteils 18 ausgeschlossen ist. Insbesondere muß zum Lösen des zweiten Gehäuseteils 18 das Halteelement 19 gegen Federkraft eingedrückt werden. Zum Einsetzen und/oder Auswechseln des Reservoirs 3 ist das zweite Gehäuseteil 18 vom Zerstäuber 1 lösbar. Das zweite Gehäuseteil 18 bildet vorzugsweise ein kappenartiges Gehäuseunterteil und/oder um- bzw. übergreift einen unteren freien Endbereich des Reservoirs 3.

Das zweite Gehäuseteil 18 kann relativ zum ersten Gehäuseteil 16 gedreht werden, wobei das Innenteil 17 mitgedreht wird. Dadurch wird die Antriebsfeder 7 über ein nicht im einzelnen dargestelltes, auf die Halterung 6 wirkendes Getriebe in axialer Richtung gespannt. Mit dem Spannen wird das Reservoir 3 axial nach unten bzw. mit seinem Endbereich (weiter) in das zweite Gehäuseteil 18 bzw, zu dessen stirnseitigem Ende hin bewegt, bis das Reservoir 3 eine in Fig. 2 angedeutete Endlage einnimmt. In diesem Zustand ist die Antriebsfeder 7 gespannt.

Der Zerstäuber 1 weist vorzugsweise eine Einrichtung zur zwangsweisen Belüftung des Reservoirs 3, insbesondere einer Außenhülle 23 des Reservoirs 3, auf.

Insbesondere kann beim erstmaligen Spannen ein beispielsweise bodenseitiges Anstechen bzw. Öffnen des Reservoirs 3 bzw. der Außenhülle 23. Beim Darstellungsbeispiel kommt eine axial wirkende, im Gehäuseteil 18 angeordnete Feder 20 am Reservoirboden 21 zur Anlage, die mit einem Anstechelement 22 eine bodenseitige, insbesondere gasdichte Versiegelung bei der erstmaligen Anlage zur Belüftung ansticht.

Die Einrichtung zur zwangsweisen Belüftung ist hier also durch das Anstechelement 22 gebildet, das von der Feder 20 gehalten oder gebildet ist. Jedoch sind auch andere konstruktive Lösungen möglich.

Es ist anzumerken, daß bei dem Anstechen zur Belüftung lediglich die Außenhülle 23 des Reservoirs 3 geöffnet wird. Der Fluidraum 4 (Beutel) mit dem Fluid 2 bzw. die Wandung 25 bleibt unbeschädigt.

Bei der Entnahme des Fluids 2 über das Förderelement kollabiert der flexible bzw. verformbare Beutel bzw. Fluidraum 4 bzw. wird die Wandung 25 verformt. Zum Druckausgleich kann die Umgebungsluft über die Belüftungs- bzw. Anstechöffnung in das Reservoir 3 bzw. die Außenhülle 23 nachströmen.

Die Einrichtung zur zwangsweisen Belüftung ist nur optional vorgesehen. Insbesondere kann die Einrichtung zur zwangsweisen Belüftung ganz entfallen, beispielsweise wenn die Außenhülle 23 des Reservoirs 3 ohnehin nicht zumindest im wesentlichen gasdicht ausgebildet ist und/oder wenn eine sonstige Einrichtung, wie ein Ventil oder eine bereits offene Belüftungsöffnung, zur Belüftung vorgesehen ist.

Zur Benutzung des Zerstäubers 1 muß zunächst das Reservoir 3 eingesetzt werden. Dies erfolgt vorzugsweise dadurch, daß das zweite Gehäuseteil 18 entfernt bzw, abgezogen wird, Anschließend wird das Reservoir 3 in das Innenteil 17 axial eingeführt bzw, eingeschoben. Hierbei erfolgt ein kopfseitiges Öffnen bzw. Anschließen. Dies erfolgt durch das Förderelement, also das Förderrohr 9, das eine vorzugsweise vorgesehene, insbesondere end- oder kopfseitige Versiegelung des Reservoirs 3 durchsticht und/oder anschließend durch einen insbesondere kopfseitigen Verschluß bzw. Anschluß 24, vorzugsweise mit einem Septum hindurch in das Innere des Reservoirs 3 bzw. Fluidraums 4 eingeführt wird. So wird die fluidische Verbindung zwischen dem Reservoir 3 - genauer gesagt zwischen dem Fluidraum 4 im Reservoir 3 - über das Förderrohr 9 zum Druckerzeuger 5 bzw. zur Druckkammer 11 hergestellt.

Anschließend wird das zweite Gehäuseteil 18 wieder aufgesetzt bzw. aufgeschoben. Nun kann das erstmalige Spannen des Zerstäubers 1 erfolgen. Vorzugsweise wird hierbei dann das Reservoir 3 durch das Anstechelement 22 bodenseitig angestochen, also zwangsweise belüftet, wir bereits erläutert.

Vor der erstmaligen Benutzung erfolgt nach dem Einsetzen bzw. fluidischen Anschließen des Reservoirs 3 ein vorzugsweise mehrmaliges Spannen und Auslösen des Zerstäubers 1. Durch dieses sogenannte Primen wird im Förderelement und/oder im Druckerzeuger 5 bis zur Austragsdüse 12 eventuell vorhandene Luft von dem Fluid 2 verdrängt. Anschließend ist der Zerstäuber 1 zur Ausgabe bzw. Inhalation bereit.

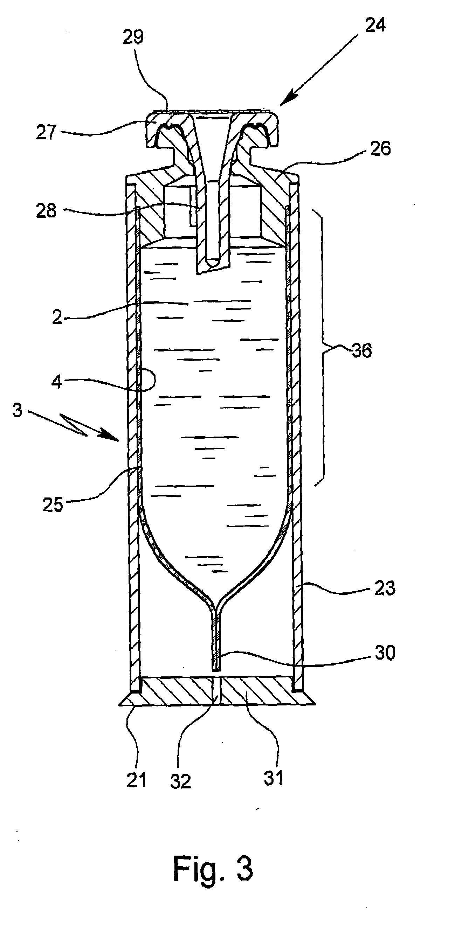

Fig. 3 zeigt in einem schematischen Schnitt einen besonders bevorzugten Aufbau des Reservoirs 3. Es ist anzumerken, daß die Darstellung nicht maßstabsgerecht ist. Das Reservoir 3 bzw. dessen Anschluß / Verschluß 24 weist beim Darstellungsbeispiel vorzugsweise ein Anschlußstück 26 auf, das mit der Außenhülle 23 und/oder Wandung 25 verbunden ist. Weiter ist vorzugsweise ein Verschlußteil 27 vorgesehen, das mit dem Anschlußstück 26 vorzugsweise gasdicht verbunden ist und insbesondere ein von dem Förderelement durchstechbares Septum 28 aufweist bzw. bildet. Das Verschlußteil 27 bzw. dessen Einführöffnung kann zusätzlich durch eine optionale Versigelung 29 verschlossen sein, die durch das Förderelement öffenbar ist, insbesondere durchstechbar ist.

Beim Darstellungsbeispiel wird der Verschluß bzw. Anschluß 24, also vorzugsweise durch das Anschlußstück 26 und das Verschlußteil 27 sowie optional aus der Versiegelung 29 gebildet, Jedoch sind auch andere konstruktive Lösungen möglich. Der gezeigte Aufbau stellt lediglich eine bevorzugte Ausführung dar.

Der Verschluß bzw. Anschluß 24 verschließt das Reservoir 3 bzw. den Fluidraum 4 an einem ersten Ende, insbesondere in einem Kopfbereich bzw. stirnseitig.

Der Verschluß bzw. Anschluß 24 bzw. dessen Anschlußstück 26 ist fest und vorzugsweise dicht mit dem Fluidraum 4 bzw. der Außenhülle 23 und/oder Wandung 25 verbunden.

Der Fluidraum 4 bzw, die Wandung 25 ist insbesondere in einem dem ersten Ende bzw. Verschluß / Anschluß 24 gegenüberliegenden Endbereich 30 verschlossen, insbesondere durch Verschweißen der Wandung 25. Jedoch sind hier auch andere konstruktive Lösungen möglich.

Das Reservoir 3 bzw. die Außenhülle 23 ist beim Darstellungsbeispiel an dem dem Verschluß bzw. Anschluß 24 entgegengesetzten Ende vorzugsweise mit einem Bodenstück 31 versehen. Vorzugsweise verschließt das Bodenstück 31 die Außenhülle 23 endseitig bzw. stirnseitig.

Vorzugsweise ist das Bodenstück 31 mit der Außenhülle 23 fest verbunden, insbesondere an diese angespritzt oder auf sonstige geeignete Weise, beispielsweise durch Verkleben, Verschweißen und/oder Klemmen mit dieser verbunden bzw. von dieser gehalten.

Das Bodenstück 31 bildet den Reservoirboden 21. Das Bodenstück 31 weist beim Darstellungsbeispiel vorzugsweise eine Belüftungsöffnung 32 auf. Die Belüftungsöffnung 21 kann bedarfsweise im Lagerzustand des Reservoirs 3 versiegelt sein (nicht dargestellt). Diese Versiegelung der Belüftungsöffnung 32 kann dann durch die bereits erwähnte Einrichtung zur zwangsweisen Belüftung beim Einsetzen des Reservoirs 3 in den Zerstäuber 1 und/oder bei der erstmaligen Benutzung oder zu einem sonstigen Zeitpunkt - insbesondere durch Anstechen - geöffnet werden.

Die Außenhülle 23 ist vorzugsweise zumindest im wesentlichen starr, länglich und/oder zylindrisch bzw. hohlzylindrisch, insbesondere hülsenartig, ausgebildet.

Die Außenhülle 23 verbindet vorzugsweise den Verschluß / Anschluß 24 bzw. das Anschlußstück 26 einerseits und das Bodenstück 31 andererseits ausreichend starr, um das Reservoir 3 durch entsprechenden Druck auf den Reservoirboden 21 bzw. das Bodenstück 31 in den Zerstäuber 1 einsetzen zu können und/oder um das Reservoir durch Ziehen am Bodenstück 31 bzw. dessen Rand wieder aus dem Zerstäuber 1 unter Lösen von der Halterung 6 herausziehen zu können.

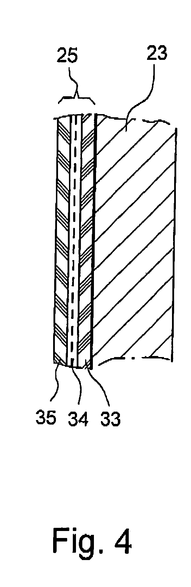

Fig. 4 zeigt in einem ausschnittsweisen vergrößerten schematischen Schnitt einen bevorzugten Wandaufbau des Reservoirs 3. In dem gezeigten Ausschnitt liegt die Wandung 25 innen an der Außenhülle 23 an.

Die Wandung 25 ist zumindest im wesentlichen oder teilweise verformbar bzw. flexibel ausgebildet, um ein möglicht leichtes Kollabieren des Fluidraums 4 bei der Entnahme von Fluid 2 zu gestatten. Der Fluidraum 4 ist nämlich zumindest weitestgehend gasdicht abgeschlossen. Die Wandung 25 ist dementsprechend weitestgehend gasdicht ausgebildet.

Die Wandung 25 ist vorzugsweise mehrschichtig aufgebaut, insbesondere weist die Wandung 25 eine Außenschicht 33, eine vorzugsweise metallische Sperrschicht 34 (insbesondere eine Metallfolie) und eine Innenschicht 35 sowie gegebenenfalls weitere Schichten auf. Die einzelnen Schichten können beispielsweise durch Beschichten, Auflaminieren oder auf sonstige geeignete Art und Weise gebildet werden. Die Sperrschicht ist insbesondere als Aluminiumschicht oder -folie ausgeführt.

Die Wandung 25 bzw. deren Schichtaufbau ist vorzugsweise folienartig ausgebildet bzw. aus Folienmaterial hergestellt.

Vorschlagsgemäß wird die Außenhülle 23 - zumindest bereichsweis, vorzugsweise über die gesamte Länge- auf die Wandung 25, also von außen, extrudiert. Die Außenhülle 23 liegt dann mit ihrer Innenseite unmittelbar auf der Außenseite bzw. Außenschicht 33 der Wandung 25 auf, wie dies in dem in Fig. 3 angedeuteten Anlagebereich 36 der Fall ist.

Bei der Herstellung wird aus der Wandung 25 bzw. dem die Wandung 25 bildenden Folienmaterial zunächst eine Hülse bzw. hohlzylindrische Form bzw. ein Schlauch - insbesondere durch Längsverschweißen eines entsprechenden Materialstreifens - gebildet. Hierauf wird die Außenhülle 23 insbesondere fortlaufend bzw, über Ihre gesamte Länge - besonders bevorzugt als Endlosschlauch - extrudiert. Um dabei ein Zusammenfallen der die innere Kontur der Außenhülle 23 gebenden Wandung 25 zu verhindern, werden geeignete Maßnahmen ergriffen. Beispielsweise kann der die Wandung 25 bildende Schlauch über einen geeigneten Dom gezogen und/oder durch ein geeignetes Medium, wie Gas oder Flüssigkeit, in der gewünschten Form gehalten bzw. aufgeblasen werden.

Ein wesentlicher Aspekt der vorschlagsgemäßen Lösung liegt darin, daß die Wandung 25 zumindest partiell oder insgesamt von der Außenhülle 23 wieder lösbar ist. Insbesondere verbindet sich die Außenhülle 23 nicht fest mit der Wandung 25. Beim Darstellungsbeispiel wird diese Lösbarkeit vorzugsweise durch eine entsprechende Materialpaarung erreicht. Die Außenhülle 23 wird aus einem ersten Material hergestellt, das sich mit der Außenseite bzw. Außenschicht 33 der Wandung 25 bzw. einem zweiten Material, das die Außenseite bzw. Außenschicht 33 der Wandung 25 bildet, nicht verbindet. Die beiden Materialien sind also vorzugsweise verschieden. Jedoch können grundsätzlich auch sonstige Haftverhinderer eingesetzt werden.

Bei dem ersten Material handelt es sich vorzugsweise um ein PE (Polyethylen) oder ein PET (Polyethylentherephtalat). Wenn für das erste Material PE gewählt wird, wird für das zweite Material vorzugsweise PET oder ein sonstiges sich nicht mit PE verbindendes Material gewählt. Wenn für das erste Material PET gewählt wird, wird für das zweite Material vorzugsweise PE oder ein sonstiges sich nicht mit PET verbindendes Material gewählt. Insbesondere ist also vorgesehen, daß das erste Material ein PE oder ein PET und das zweite Material dann ein PET oder PE ist.

Durch die vorgenannte oder eine sonstige Materialpaarung wird also bevorzugt erreicht, daß sich die beiden Materialien beim Aufextrudieren der Außenhülle 23 nicht unlösbar miteinander verbinden.

Nach dem Aufextrudieren der Außenhülle 23 und einem eventuell erforderlichen Ablängen bei Endlosherstellung werden die hülsenartigen Stücke verschlossen, um das Reservoir 3 bzw, den Fluidraum 4 zu bilden.

Vorzugsweise wird zunächst der Anschluß 24 bzw. das Anschußstück 26 angebracht, besonders bevorzugst angespritzt. Hierzu wird vorzugsweise wiederum ein geeignetes Material, wie PE oder PET, das sich mit der Wandung 25 und/oder Außenhülle 23 verbindet, für das Anschlußstück 26 gewählt. Beispielsweise kann sich das Anschlußstück 26 direkt mit der Innenseite der Wandung 25 und der stirnfläche der Außenhülle 23 verbinden. Alternativ oder zusätzlich kann das Anschlußstück 26 auch die Außenhülle 23 außenseitig übergreifen und/oder sich mit einem axial gegenüber dem Ende der Wandung 25 vorspringenden Bereich der Außenhülle 23 - also der Innenseite der Außenhülle 23 - verbinden.

Vorzugsweise ist die Wandung 25 nur mit dem Anschlußstück 26 fest oder unlösbar verbunden.

Die vorgenannten Ausführungen bezüglich der Verbindungsmöglichkeiten des Anschlußstücks 26 mit der Außenhülle 23 und/der Wandung 25 gelten vorzugsweise auch entsprechend für den Verschluß bzw. Anschluß 24, insbesondere auch bei einem sonstigen Aufbau des Verschlusses bzw. Anschlusses 24.

Nach dem bevorzugten Anspritzen des Anschlußstücks 26 bzw. dem Anbringen des Verschlusses / Anschlusses 24 kann das Verschließen des Reservoirs 3 bzw. Fluidraums 4 am gegenüberliegenden Ende - insbesondere im Endbereich 30 - erfolgen. Jedoch kann dies bedarfsweise auch zuerst, also vor dem Anbringen des Anschlußstücks 26 bzw. Verschlusses / Anschlusses 24 erfolgen.

Zum Verschließen des Fluidraums 4 wird die Wandung 25 im Endbereich 30 verschlossen, insbesondere durch innenseitiges Verschweißen. Hierzu wird die Außenhülle 23 besonders bevorzugt von außen derart zusammengepreßt bzw. flachgedrückt, daß die Wandung 25 entsprechend innenseitig zusammengepreßt und dann durch entsprechende Energiezufuhr oder auf sonstige Weise innenseitig im Endbereich 30 miteinander verbunden bzw. verschweißt wird. Dadurch wird der Fluidraum 4 in Endbereiche 30 dicht abgeschlossen.

Anschließend kann die Außenhülle 23 wieder ihre vorzugsweise zumindest im wesentlichen hohlzylindrische Form auch im flachgedrückten Bereich annehmen, insbesondere aufgrund ihrer Eigenelastizität oder durch sonstige Rückstellung. Bei dieser Rückstellung löst sich dann die Wandung 25 zumindest in diesem Bereich von der Außenhülle 23. Der verschlossene und im wesentlichen flachgedrückte Endbereich 30 der Wandung 25 wird dann von der Außenhülle 23 auf deren Innendurchmesser verformt bzw. zusammengedrückt oder gefaltet.

Der sich dann ergebenen Zustand ist zumindest im wesentlichen schematisch in Fig. 3 dargestellt. Die zunächst bei der Herstellung über die gesamte Länge an der Außenhülle 23 anliegende Wandung 25 liegt dann anfänglich (ausgehend von dem mit Fluid 2 bereits oder noch gefülltem Reservoir 3 bzw. Fluidraum 4) zunächst noch in einem in Fig. 3 schematisch angedeuteten Anlagebereich 36 an der Innenseite der Außenhülle 23 an. Mit zunehmender Entnahme von Fluid 2 kollabiert der von der Wandung 25 gebildete Innenbeutel bzw. Fluidraum 4, so daß sich dementsprechend der Anlagebereich 36 dann verkleinert. Die Wandung 25 löst sich also nicht nur bei der Herstellung des Reservoirs 3, sondern auch bei der Entnahme von Fluid 2 aus dem Reservoir 3 von der Innenseite der Außenhülle 23, zumindest bereichsweise.

Das Füllen des Reservoirs 3 bzw, des Fluidraums 4 mit dem Fluid 2 kann wahlweise entweder über den Verschluß 24 - also das eigentliche Entnahmeende - oder über das andere, hier bodenseitige Ende erfolgen. Im ersten Fall wird das Fluid 2 vor dem Verschließen des Verschlusses 4, insbesondere vor dem Einsetzen des Verschlußteils 27 mit dem Fluid 2 gefüllt (das Verschlußteil 27 wird vorzugsweise gasdicht mit dem Anschlußstück 26 verschweißt, um den Verschluß 24 zu verschließen). Im zweiten Fall wird das Fluid 2 vor dem Verschließen der Wandung 25 im Endbereich 30 eingefüllt, wobei dann vorher der Verschluß 24 am anderen Ende des Reservoirs 3 angebracht und verschlossen wurde.

Das Verschließen der Außenhülle 30 am bodenseitigen Ende durch das Bodenstück 31 erfolgt nach dem Verschließen des Fluidraums 4 bzw. der Wandung 25 im Endbereich 30. Das Bodenstück 31 kann beispielsweise durch Klemmen, Kleben, Anspritzen und/oder Schweißen oder auf sonstige Art und Weise mit der Außenhülle 23 insbesondere unlösbar verbunden werden.

Wie bereits erwähnt, erfolgt die Herstellung bzw, das Aufbringen der Außenhülle 23 vorschlagsgemäß durch Extrudieren auf die Wandung 25, die den Fluidraum 4 bzw. Innenbeutel bildet. Unter dem Begriff "Extrudieren" ist in einem engeren Sinne vorzugsweise die Herstellung der Außenhülle 23 in einem Endlosverfahren bzw. kontinuierlich und/oder die Erzeugung der Außenhülle 23 über eine Flach- oder Hohldüse und/oder ein entsprechendes Anformen zu verstehen. In einem weiteren Sinne soll unter dem Begriff "Extrudieren" aber auch ein sonstiges, ggf. auch diskontinuierliches Anspritzen, Anformen bzw. Spritzgießen der Außenhülle 23 auf die Wandung 25 zu verstehen sein.

Insbesondere können das vorschlagsgemäße Reservoir 3 und die vorliegende Erfindung generell auch bei den Zerstäubern bzw. Inhalern, die in den nachfolgend genannten Druckschriften beschrieben sind oder auf deren Prinzipien beruhen, verwendet werden: EP 1236 517, EP 1 561 484, EP 1 562 094, EP 1 604 701, JP 2004-0283245, JP 2004-249208, JP 2004-283244, JP 2003/058421, US 2002/0153006, US 2003/0100964, US 2003/0127538, US 2004/0163646, US 2005/0034723, US 2005/0133029, US 2005/0172957, US 2005/0224076, US 2005/0268911, US 5,915,378,WO 03/041774, WO 2004/022128, WO 2004/039442, WO 2004/078244,

Vorzugsweise handelt es sich bei dem Fluid 2 um eine Flüssigkeit, wie bereits erwähnt, insbesondere um eine wäßrige oder ethanolische Arzneimittelformulierung. Es kann sich jedoch auch um eine sonstige Arzneimittelformulierung, eine Suspension oder dgl. oder auch um Partikel oder Pulver handeln.

Nachfolgend werden bevorzugte Bestandteile, Verbindungen und/oder Formulierungen des vorzugsweise medizinischen Fluids 2 aufgeführt. Wie bereits erwähnt, kann es sich um wäßrige oder nicht wäßrige Lösungen, Mischungen, ethanolhaltige oder lösungsmittelfreie Formulierungen o. dgl. handeln. Besonders bevorzugt sind im Fluid 2 enthalten:

Die unten genannten Verbindungen können allein oder in Kombination zur Anwendung in der erfindungsgemäßen Vorrichtung gelangen. In den unten genannten Verbindungen ist **W** einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmem, Dopamin-Agonisten, H1-Antihistaminika, PAF-Antagonisten und PI3-Kinase Inhibitoren. Weiterhin können zwei- oder dreifach Kombinationen von **W** kombiniert werden und zur Anwendung in der erfindungsgemäßen Vorrichtung gelangen. Beispielhaft genannte Kombinationen von **W** wären:
- **W** stellt ein Betamimetikum dar, kombiniert mit einem Anticholinergikum, Corticosteroide, PDF4-Inhibitore, EGFR-Hemmern oder LTD4-Antagonisten,
- **W** stellt ein Corticosteroid dar, kombiniert mit einem PDE4-Inhibitor, EGFR-Hemmern oder LTD4-Antagonisten
- **W** stellt einen PDE4-Inhibitor dar, kombiniert mit einem EGFR-Hemmer oder LTD4-Antagonisten
- **W** stellt einen EGFR-Hemmer dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HO-KU-81, KUL-1248 und
- 3-(4-{6-[2-Hydroxy-2-(4-hydroxy-3-hydroxymethyl-phenyl)-ethylamino]-hexyloxy}-butyl)-benzyl-sulflonamid
- 5-[2-(5,6-Diethyl-indan-2-ylamino)-1-hydroxy-ethyl]-8-hydroxy-1H-quinoliun-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-(3-(4-Methoxybenzyl-amino)-4-hydroxyphenyl)-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylaminojethanoi
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethylethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethylethylamino]-ethyl}-4H-benzo[1,4]oxazin-3-on
- 8-{2-[1,1-Dimethyl-2-(2,4,6-trimeylphenyl)-ethylamino]-1-hydroxyethyl}-6-hydroxy-4H-benzo[1,4]oxazin-3-on
- 6-Hydroxy-8-{1-hydroxy-2-[2-(4-hydroxy-phenyl)-1,1-dimethylethylamino]-ethyl}-4H-benzo[1,4]oxazin-3-on
- 6-Hydroxy-8-{1-hydroxy-2-[2-(4-isopropyl-phenyl)-1,1dimethylethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylammo]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert,-butylamino)eanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylaamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3,(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydxoxy-2-(4-hydroxy-3-hydroxymethyl-phenyl)-ethylamino]-heptyloxy}-propyl)-benzylsulfonamid
- 4-(2-{6-[4-(3-Cyclopentanesulibnyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethylphenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC-1** 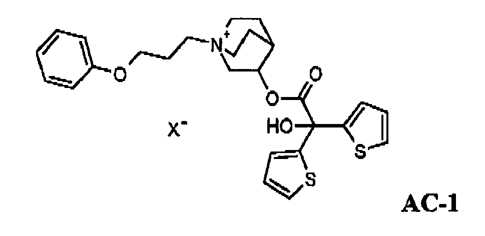 worin X ⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 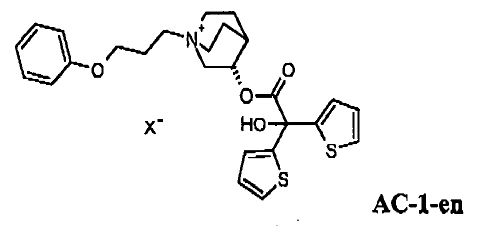 enthalten, worin X ⁻ die vorstehend genannten Bedeutungen aufweisen kann. Weiterhin bevorzugte Anticholinergika sind ausgewählt aus den Salzen der Formel **AC-2** 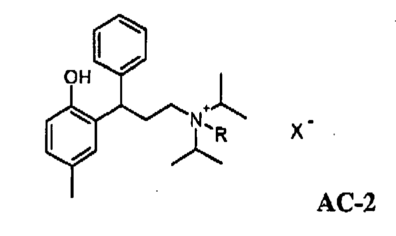 worin R entweder Methyl oder Ethyl bedeuten und worin X ⁻ die vorstehend genannte Bedeutungen aufweisen kann. In einer alternativen Ausführungsform kann die Verbindung der Formel **AC-2** auch in Form der freien Base **AC-2**-base vorliegen.

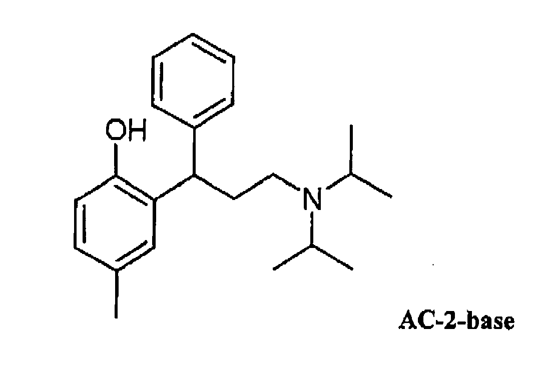

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäureüopenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobzomid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17a-(2,2,3,3-tertamethylcyclopropylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxyphenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2- hydroxy-2-propyl)phenyl)thio)methylcyclopropan-esslgsäure,
- l-(((1(R)-3(3-(2-(2,3-Dichlorthieno[3,2-b]pyridin-5-yl)-(E)-ethenyl)phenyl)-3-(2-(1-hydroxy-1,methylethyl)phenyl)-propyl)thio)methyl)cyclopropanessigsäure
- [2-[[2-(4-tert-Butyl-2-thiazolyl)-5-benzofuranyl]oxymethyl]phenyl]-essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chler-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-butcn-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methylamino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyioxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethylamino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methylamino]-1-oxo-2-buten-1-yl}amino)-7-cycbpropylmethoxy-chinazolin
- 4-[(R)-(1-Phenyl-ethyl)amino]-6-({4-[N-(tetrahydropyran-4-yl)-N-methylamino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methylamino]-1-oxo-2-buten-1-yl}amino)-7-cyclopentyloxy-chinazolin
- 4-[(3-Chlor-4-fluorphenyl)amino]-6-{[4-(N-cyclopropyl-N-methylamino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo.2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinylcarbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-ffuorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-l-yl]amino}-7-methoxy-chinazolin
- 4-[(3-Chlor-4-fluorphenyl)amino]-6-{[4-(morpholin-4-yl)-1-oxo-2-buten-1-yl]animo}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-(3-Chlor-4-fluor-phenyl)arnino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexm-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-mefihoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxyl-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethylamino)carbonyl]-piperidin-4-yloxy}-7-methoxy-chinazolin
- 4-[(3-Chtor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-pipenidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazoa
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-*N*-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1003 478 A1 oder CA 2297174 A1 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkomalkaloide: Dihydroergotamin, Ergotamin.

### Bezugszeichenliste

- 1: Zerstäuber
- 2: Fluid
- 3: Reservoir
- 4: Fluidraum
- 5: Druckerzeuger
- 6: Halterung
- 7: Antriebsfeder
- 8: Sperrelement
- 9: Förderrohr
- 10: Rückschlagventil
- 11: Druckkammer
- 12: Austragsdüse
- 13: Mundstück
- 14: Aerosol
- 15: Zuluftöffnung
- 16: Gehäuseoberteil
- 17: Innenteil
- 17a: oberes Teil des Innenteils
- 17b: unteres Teil des Innenteils
- 18: Gehäuseteil (Unterteil)
- 19: Halteelement
- 20: Feder (im Gehäuseunterteil)
- 21: Reservoirboden
- 22: Anstechelement
- 23: Außenhülle
- 24: Verschluß / Anschluß
- 25: Wandung
- 26: Anschlußstück
- 27: Verschlußteil
- 28: Septum
- 29: Versiegelung
- 30: Endbereich
- 31: Bodenstück
- 32: Belüftungsöffnung
- 33: Außenschicht
- 34: Sperrschicht
- 35: Innenschicht
- 36: Anlagebereich

## Patentansprüche

1. Reservoir (3), insbesondere für einen Zerstäuber (1), mit einer Außenhülle (23) und einem Fluidraum (4) mit einem Fluid (2), insbesondere einer Arzneimittelformulierung, wobei der Fluidraum (4) zumindest bereichsweise eine flexible oder verformbare Wandung () aufweist, so daß der Fluidraum (4) bei der Entnahme von Fluid (2) kollabiert und/oder einen verformbaren Innenbehälter bildet,
**dadurch gekennzeichnet,**
**daß** die Außenhülle (23) auf die Wandung (25) extrudiert ist und die Wandung (25) zumindest partiell bei der Entnahme von Fluid (2) von der Außenhülle (23) lösbar ist.

2. Reservoir nach Anspruch 1, **dadurch gekennzeichnet, daß** die Außenhülle (23) zumindest im wesentlichen starr, länglich und/oder zylindrisch bzw. hohlzylindrisch ausgebildet ist.

3. Reservoir nach Anspruch 1 oder 2, **dadurch gekennzeichnet, daß** die Außenhülle (23) aus einem ersten Material hergestellt ist und die Wandung (25) eine Außenseite oder Außenschicht (33) aus einem zweiten Material aufweist, wobei sich die beiden Materialien beim Aufextrudieren der Außenhülle (23) nicht unlösbar miteinander verbinden.

4. Reservoir nach Anspruch 3, **dadurch gekennzeichnet, daß** das erste Material und das zweite Material verschieden sind und/oder daß das erste Material ein PE oder ein PET und das zweite Material dann ein PET oder PE ist.

5. Reservoir nach einem der voranstehenden Ansprüche, **dadurch gekennzeichnet, daß** das Reservoir (3) ein Anschlußstück (26) aufweist, insbesondere wobei das Anschlußstück (26) an die Außenhülle (23) und/oder Wandung (25) angespritzt oder auf sonstige Weise damit fest verbunden ist.

6. Resrvoir nach Anspruch 5, **dadurch gekennzeichnet, daß** die Wandung (25) nur mit dem Anschlußstück (26) fest oder unlösbar verbunden ist.

7. Reservoir nach einem der voranstehenden Ansprüche, **dadurch gekennzeichnet, daß** das Reservoir (3) ein Bodenstück (31) aufweist, das mit der Außenhülle (23) verbunden ist, insbesondere durch Klemmen, Kleben, Anspritzen und/oder Schweißen.

8. Reservoir nach einem der voranstehenden Ansprüche, **dadurch gekennzeichnet, daß** der Fluidraum (4) zumindest teilweise oder im wesentlichen beutel- oder schlauchartig ausgebildet ist.

9. Reservoir nach einem der voranstehenden Ansprüche, **dadurch gekennzeichnet, daß** die Wandung (25) folienartig ausgebildet ist.

10. Reservoir nach einem der voranstehenden Ansprüche, **dadurch gekennzeichnet, daß** die Wandung (25) mehrschichtig ausgebildet ist, insbesondere eine metallische Sperrschicht (34) und/oder eine Außenschicht (33) aus Kunststoff aufweist.

11. Reservoir nach einem der voranstehenden Ansprüche, **dadurch gekennzeichnet, daß** das Reservoir (3) einen dem Fluidraum (4) zugeordneten Anschluß oder Verschluß (24) aufweist, der vorzugsweise durch Anschließen oder Einführen eines Förderelements, wie eines Förderrohrs (9), zur Entnahme von Fluid (2) aus dem Reservoir (3) offenbar ist.

12. Zerstäuber (1) für ein Fluid (2), vorzugsweise Inhalator, insbesondere zur medizinischen Aerosol-Therapie, mit einem Reservoir (3) mit einer Außenhülle (23) und einem Fluidraum (4) mit einem Fluid (2), insbesondere einer Arzneimittelformulierung, wobei der Fluidraum (4) zumindest bereichsweise eine flexible oder verformbare Wandung (25) aufweist, so daß der Fluidraum (4) bei der Entnahme von Fluid (2) kollabiert und/oder einen verformbaren Innenbehälter bildet,
**dadurch gekennzeichnet,**
**daß** das Reservoir (3) gemäß einem der voranstehenden Ansprüche ausgebildet ist.

13. Verfahren zum Herstellen eines Reservoirs (3), insbesondere für einen Zerstäuber (1), wobei ein Fluidraum (4) für ein Fluid (2), insbesondere eine Arzneimittelformulierung, aus einer folienartigen, flexiblen und/oder verformbaren Wandung (25) gebildet wird,
**dadurch gekennzeichnet,**
**daß** eine Außenhülle (23) auf die Wandung (25) extrudiert wird, wobei die Wandung (25) zumindest partiell von der Außenhülle (23) wieder lösbar ist.

14. Verfahren nach Anspruch 13, **dadurch gekennzeichnet, daß** die Wandung (25) zunächst eine zumindest im wesentlichen zylindrische Außenkontur bildet, auf die die Außenhülle (23) extrudiert wird.

15. Verfahren nach Anspruch 13 oder 14, **dadurch gekennzeichnet, daß** die zumindest im wesentlichen hohlzylindrische Außenhülle (23) temporär bereichsweise flach gedrückt wird, um die Wandung (25) innenseitig und/oder an einem Endbereich (30) zu verschweißen, und die Außenhülle (23) dann unter Lösen von der Wandung (25) wieder ihre zumindest im wesentlichen hohlzylindrische Form annimmt.
